# EUROPEAN PATENT SPECIFICATION

(11) **EP 3 853 603 B1**
(45) Date of publication and mention of the grant of the patent: **25.12.2024**
(21) Application number: 19766268.7
(22) Date of filing: 17.09.2019
(51) Int. Cl.: G01N 33/00, G01N 31/00, A61B 5/00

(54) **DEVICE FOR MEASURING WATER CONTENT**
GERÄT ZUR MESSUNG DES WASSERGEHALTS
DISPOSITIF DE MESURE DE LA TENEUR EN EAU

(30) Priority: 17.09.2018 EP 18194729
(43) Date of publication of application: 28.07.2021
(73) Proprietor: Epymetrics AG, 8055 Zürich (CH)
(72) Inventor: HEEB, Peter, 9436 Balgach (CH); BAUR, Monika, 9326 Horn (CH); CAGIN, Emine, 9470 Buchs (CH); BERNARD, André, 9014 St. Gallen (CH)
(74) Representative: Kasche & Partner
(86) International application number: PCT/EP2019/074784
(87) International publication number: WO 2020/058232

(56) References cited:
- DE-A1- 102016 212 948
- JP-A- 2018 079 298
- ZEYI ZHOU ET AL: "Determination of micro-traces of water in gases by a hydrogen gas sensor combined with a conversion reactor column; Determination of micro-traces of water in gases by a hydrogen gas sensor combined with a conversion reactor column", MEASUREMENT SCIENCE AND TECHNOLOGY, IOP, BRISTOL, GB, vol. 15, no. 10, 15 September 2004 (2004-09-15), pages 2170 - 2174, XP020066399, ISSN: 0957-0233, DOI: 10.1088/0957-0233/15/10/030
- F. E. HARRIS ET AL: "Determination of Traces of Water Vapor in Gases", ANALYTICAL CHEMISTRY, vol. 23, no. 5, May 1951 (1951-05-01), US, pages 736 - 739, XP055574460, ISSN: 0003-2700, DOI: 10.1021/ac60053a015
- KULKARNI A ET AL: "Fabrication and characterization of innovative gas flow sensor", SENSORS AND ACTUATORS A: PHYSICAL, ELSEVIER BV, NL, vol. 122, no. 2, 26 August 2005 (2005-08-26), pages 231 - 234, XP027807131, ISSN: 0924-4247, [retrieved on 20050826]

## Description

### BACKGROUND

High-precision measurement of water content in the flow regime of lessthan 10 microliter/minute iscrucial for many applications. Today, there are multiple state-of-the art solutions; however, each suffers from a deficiency. Many solutions require expensive, specialized equipment that are dependent on an external electrical power source and require a lab setting. Examples include, thermal massflow meter, Doppler ultrasonic flow meters, Venturi flow meters, Coriolisflow metersjust to name a few.

Thermal mass-flow meters, also called thermal anenometers, are simpler to employ. However, they are limited to a defined continuum of flow of fluid having a constant and well-known density and heat capacitance within the volume stream passing the sensor. Thermal anenometers are therefore incapable of measuring water flow in, for example, aqueous solutions of unknown composition exhibiting unknown thermo-physical parameters.

Therefore, there is a need for a high-precision measurement device that can reliably and precisely determine the flow rate of an aqueous solution, circulating in a known environment (such as a microfluidic system), that is inexpensive, and deployable in a wide variety of settings function in the above-noted flow regime.

ZEYI ZHOU ET ALdiscloses: "Determination of micro-traces of water in gases by a hydrogen gas sensor combined with a conversion reactor column; Determination of micro-traces of water in gases by a hydrogen gas sensor combined with aconversion reactor column", MEASUREMENT SCIENCE AND TECHNOLOGY, vol. 15, no. 10, 15 September 2004 (2004-09-15), pages2170-2174, XP020066399, ISSN: 0957-0233, DOI: 1 0.1 088/0957-0233/15/10/030, discloses: Micro-traces of water in gases have been determined by using a combination of a conversion reactor column and a high sensitivity hydrogen gas sensor (abbreviated here to CRHSGC). A special purge installation was designed to clean the system with dried and purified liquid nitrogen. Micro-traces of water in gases first were introduced into the reactor column. The column was prepared by filling with a mixture of lithium and aluminium hydride (LiAIH4) and glass fibre in a vacuum box, where the water present reacted with LiAIH4 at about 60 °C. Hydrogen was released and determined by the hydrogen gas sensor, which was calibrated by a series of standard micro-trace hydrogen mixture gases. The experimental results showed that this method can be used to determine micro-traces of water in gases quickly, accurately and at a low cost. Only about 2-5 litresof sample were needed during each analysis. The method developed does not have the shortcoming of direct determination of micro-traces of water in gases that is caused by the absence of appropriate traceable calibration gases.

JP 2018-79298 A discloses skin moisture content measuring devices.

### BRIEF DESCRIPTION OF THE DRAWINGS

The figures illustrate generally, by way of example, but not by way of limitation, various embodiments discussed in the present document.
For simplicity and clarity of illustration, elements shown in the figures have not necessarily been drawn to scale. For example, the dimensions of some of the elements may be exaggerated relative to other elements for clarity of presentation. Furthermore, reference numerals may be repeated among the figures to indicate corresponding or analogous elements. References to previously presented elements are implied without necessarily further citing the drawing or description in which they appear. The number of elements shown in the Figures should by no means be construed as limiting and is for illustrative purposes only. The figures are listed below.
**FIG. 1** is a schematic, cross-sectional side-view of a measurement device, according to some embodiments;
**FIGS. 2A-2F** are schematic, cross-sectional side-views of various embodiments of a transducing element employable in the measurement device of **FIG. 1****;**
**FIG. 3** is a schematic, cross-sectional side-view of a measurement device mounted on a body to measure the flow rate of water, for example, due to the discharge of fluid from matter (e.g., perspiration or sweat rate), according to some embodiments; and
**FIG. 4** is a flowchart of the process steps employed by the measurement device, according to some embodiments.

### DETAILED DESCRIPTION

The following description sets forth various details to provide a thorough understanding of the invention and it should be appreciated, by those skilled in the art, that the present invention may be practiced without these specific details. Furthermore, well-known methods, procedures, and components have been omitted to highlight the present invention.

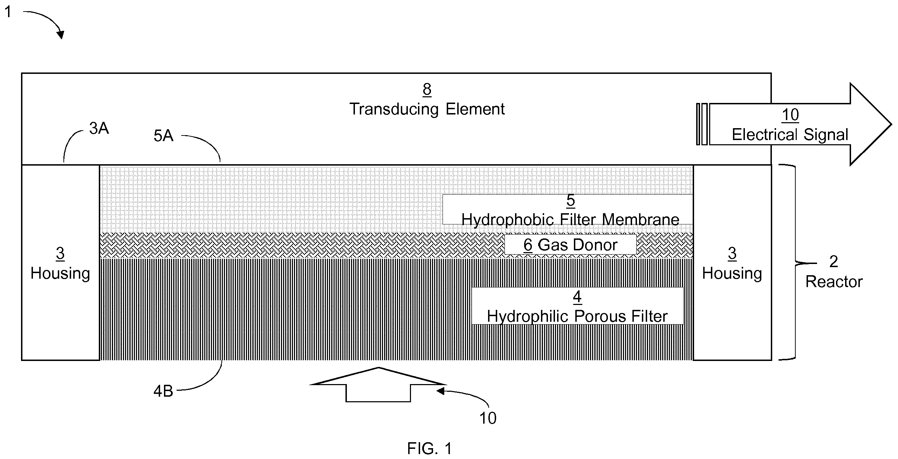

Turning now to the figures, **FIG. 1** is a schematic, cross-sectional side-view of a measurement device **1** and includes two primary elements; a reactor **2** that directly or indirectly engages with and/or is in fluid communication with transducing element **8,** according to the invention. Reactor **2** includes, for example, a (e.g., polymeric) housing **3** that may at least partially encase a hydrophilic porous filter **4** for capturing moisture and filtering impurities, a gas donor **6** characterized by its ability to generate (e.g., hydrogen) gas upon reaction with water, and a hydrophobic filter membrane **5** operative to capture unreacted water and other reactants. Transducing element **8** is operative to transduce the gas into an electrical signal **10** and can be implemented in various configurations as will be further discussed.

In some embodiments, hydrophilic porous filter **4** can be disposed such to be in fluid communication with a fluid source **10.**

In some embodiments, gas donor **6** may be disposed between hydrophilic porous filter **4** and hydrophobic filter membrane **5.** In some embodiments, hydrophobic filter membrane **5** may be disposed between gas donor **6** and transducing element **8.** Optionally, hydrophilic porous filter **4,** hydrophobic filter membrane **5,** gas donor **6** and/or transducing element **8** may be disposed in a layered manner. Optionally, housing **3** may extend laterally from an upper surface **5A** of hydrophobic filter membrane **5** to a lower surface **4B** of hydrophilic porous filter **4.** Housing **3** may therefore laterally encase hydrophilic porous membrane **4, gas donor 6** and hydrophobic filter membrane **5.** In some embodiments, as exemplified in **FIG.** 1, transducing element **8** may extend over the upper surface **5A** of hydrophobic filter membrane **5** and the upper surface or edge **3A** of housing **3.** Optionally, the upper surfaces **3A** and **5A** may be flush. In some other embodiments, the upper surfaces **3A** and **5A** may not be flush. Further, in some other embodiments, transducing element **8** may be sized such to not extend over the later edges of hydrophobic filter membrane **5.** Optionally, housing **3** may be arranged to laterally extend over the lateral edges of transducing element **8.**

The lower surface **4B** of hydrophilic porous filter **4** is exposed to allow for the continuous measurement of water content in liquid discharged by the skin of a living mammalian.

In some embodiments, measurement device **1** may comprise a fastener for allowing operably (and optionally, removably) coupling measurement device 1 with an animal body such to allow for the measurement of water content contained in bodily fluid discharged from the animal body. The fastener may include, for example, an adhesive, staple, tack, suture, and/or the like.

In accordance with the invention, measurement device **1** is configured as a patch-like structure.

In accordance with the invention, measurement device **1** is configured to be operably engaged with a skin surface portion of an animal body.

Hydrophilic porous filter **4** provides a constant flow resistance to both liquid and gas states in the above-noted flow regime, and the hydrophilic properties do not hinder passage or facilitate passage of liquid water at the low pressures that may be associated with low flow rate regimes towards gas donor **6.** For example, the hydrophilic properties of porous filter **4** may facilitate the passage of water-containing liquid from fluid source **10** towards gas donor **6.** Filter **4** may be made, for example, of glass, ceramic, metal, and/or cellulose and may, for example, have a pore size of 450 nm enabling passage of liquid and vapor while filtering particles or salt compounds.

In another embodiment, Hydrophilic porous filter **4** is implemented as Anodic Aluminum Oxide or Anodic Titanium Oxide with a pore size of, e.g., 5-500nm.

As shown, gas donor **6** is disposed at the downstream side of filter **4** to enable reaction with filtered water vapor conveyed by pressure exerted by the water source through primary porous filter **4.** In certain filters **4,** the liquid water is also conveyed through via capillary action from the outside to the inside of upstream reactor **2.**

In some embodiments, CaH₂ is employed as the gas donor. Alternative donors of hydrogen include, for example, metal-hydrides such as MgH₂, NaAlH₄, LiAlH₄, LiH, LiBH₂, LiBH4, non-metal hydrides, and/or some carbohydrates. The gas donor can act as a battery substitute and may define the upper limit of the cumulative electrical power that can be generated.

Hydrophobic filter membrane **5** is implemented as a barrier to enclose the CaH₂, H₂O and Ca(OH)₂ and prevent them from entering the downstream fuel cell, in a certain embodiment. In another embodiment, filter membrane **5** is implemented as combination cellulose/polyester cloth.

Transducing element **8** translates the gas into an electrical signal by any of a variety of transducing element embodiments, as will be now discussed.

The measurement device **1** may be configured such that hydrogen gas generated thereby is released into the environment. In this way, hydrogen gas can be continuously generated in response to (e.g., continuously) subjecting hydrophyilic porous filter **4** with (e.g., flowing) media that may contain water.

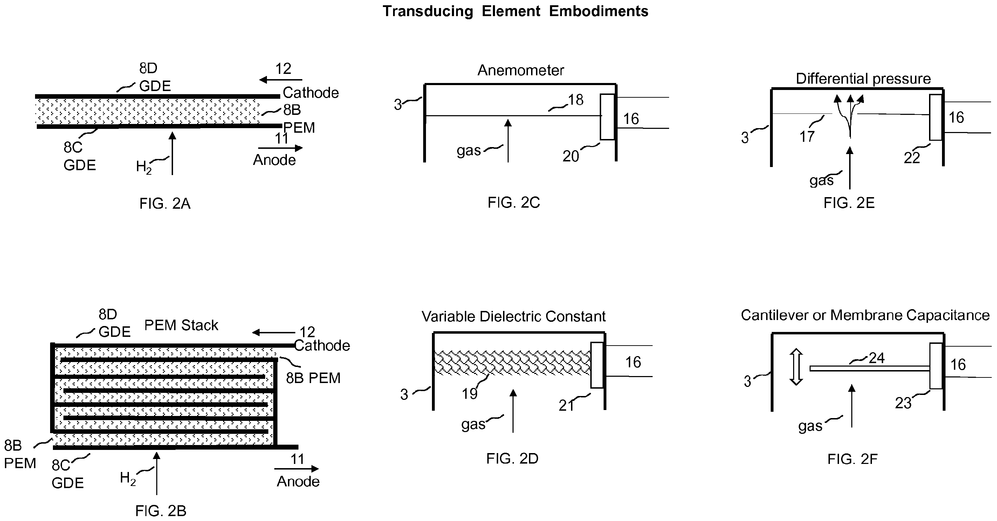

**FIGS. 2A-2F** are schematic, cross-sectional side-views of various embodiments of transducing element **8.**

Specifically, **FIG. 2A** depicts an embodiment of transducing element **8** implemented as a proton-exchange membrane (PEM) fuel cell in which hydrogen contacting a downstream gas diffusion electrode (GDE) **8C** is oxidized and the electrons exit the cell on the anode side **11** through an electrical conductor. The resulting cations traverse PEM **8B.** At GDE **8D** the hydrogen ions recombine with electrons and form water through reaction with oxygen. In a certain embodiment PEM **8B** is implemented as Nafion.

**FIG. 2B** depicts an embodiment of transducing element **8** employing a polymer stack of the PEM fuel cells.

**FIG. 2C** depicts an embodiment of transducing element **8** that employs a heating filament **18** cooled by the flow of gas. As shown, at least some of the gas is directed over a resistance heated wire **18.** The resulting change in resistance or temperature distribution profile is measured by circuitry **20** and output, e.g., via leads **16** and/or a wireless transmitter. It should be appreciated that the above noted deficiencies of an anemometer are removed through conversion of any fluid media (e.g., aqueous solution) operably engaging with the device into a pure gas. It is noted that fluid media may be characterized by its composition-dependent density and heat capacity.

**FIG. 2D** depicts an embodiment of transducing element **8** that employs a porous material **19** whose dielectric constant changes as it fills with gas. The resulting change in capacitance is processed by circuitry **21** and outputs a signal via leads **16** and/or a wireless transmitter. Zeolite is an example of a such a porous material.

**FIG. 2E** depicts an embodiment of transducing element **8** that employs a differential pressure sensor to transduce gas pressure into an electrical signal. As shown, a differential pressure is created as gas passes through orifice **17** and is transduced into an electric signal by circuitry **22,** and signal output, e.g., via leads **16** and/or a wireless transmitter.

**FIG. 2F** depicts an embodiment of transducing element **8** that employs a cantilever or stretchable membrane configured to deflect responsively to the local pressure field generated by the gas-stream. As shown, gas applies a pressure to flexible element **24** and the resulting deformation is quantified through an electromechanical transducer or circuitry **23,** and a signal output, e.g., via leads **16** and/or a wireless transmitter.

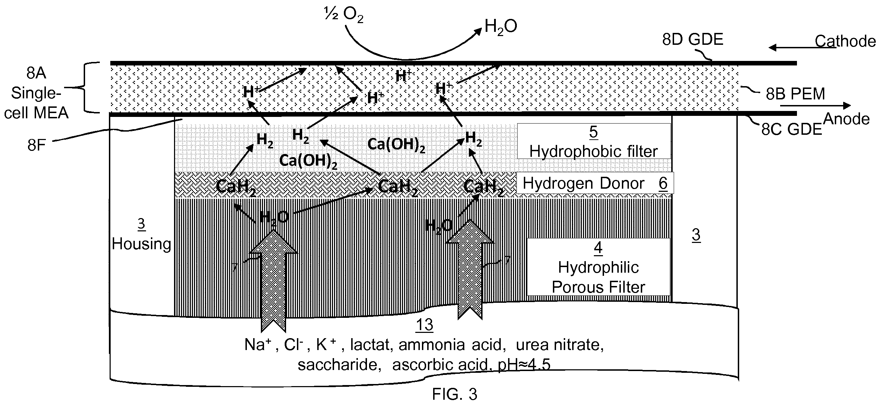

**FIG. 3** depicts an embodiment of measurement device **1** applied as a sweat gauge mounted to sweating skin **13.**

Water and other sweat constituents are captured by primary hydrophilic porous filter **4,** the non-aquatic constituents are filtered out, and the remaining water content conveyed downstream where it contacts the hydrogen donor CaH₂ **6** disposed at the downstream edge of primary filter **4.** There the CaH₂ reacts with water to form a stoichiometric volume of hydrogen that creates a pressure gradient driving the hydrogen through secondary filter **5.** Water filter **5** filters unreacted water, CaH₂ and Ca(OH)₂ and has a low flow resistance relative to primary filter **4.** This filtering may become increasingly significant in protecting transducing element **8** as reaction efficiency diminishes with time and the quantity of unreacted water increases. The hydrogen continues downstream and contacts transducing element **8** implemented in this example as a single-cell membrane electrode assembly (MEA) having a proton exchange membrane PEM **8B** sandwiched between two gas diffusion electrodes GDEs **8C** and **8D,** as noted above. Each of GDEs **8C** and **8D** has an electrically conductive supporting cloth enabling gas distribution and an electrode with a catalyst where the chemical reactions occur. The catalyst coated surfaces are in contact with PEM electrolyte **8B.**

As shown, GDE **8C** hydrogen is oxidized to cations H+ and the electrons leave measurement device **1** at anode **11.** The cations pass the solid-state electrolyte PEM **8B** and the oxygen is reduced and combines with cations to produce water at cathode **12,** as noted above.

The PEM **8B** is a gas selective permeable membrane resulting in a hydrogen and oxygen gradient across the membrane thickness. It acts as convey path for protons supply the GDE **8D** with protons H+, while blocking oxygen and ions thereof. The GDE **8D** in contact with the PEM promotes a high conversion rate of the protons to water.

MEA **8A** is in communication with gas distribution channel **8F** to maximize hydrogen contact with to the membrane surface. A high conversion rate is obtained by using MEAs. Non-converted excess hydrogen can leave the system after passing membrane surface **8D** in a certain embodiment.

In another embodiment, a bypass channel (not shown) directs a known, fixed fraction of hydrogen directly out of housing **3** and does not produce an electrical signal to prevent saturation of the fuel cell and facilitate miniaturization of MEA **8A.** Measurement device **1** can be self-actuated and deactivated in accordance with stoichiometric limitation set by the amount of water available.

As taught, the required precision measurement is achieved through the capture of sweat in static and/or any flowing state and the conversion of the sweat, based on the amount of water contained therein, into a corresponding collective electrical signal.

Furthermore, in addition to its measuring capacity, the sensor also generates harnessable electricity.

Measurement device **1** may be comparatively efficient and effective for static and non-static states and have a high sensitivity down to flow rates of the less than, for example, 10uL/min. Measurement device **1** is also effective for liquid, gaseous, and vapor states over a temperature range like, for example, 5-40°C, has a fast response time of some seconds, has a slew rate of, e.g., 3-5 seconds to reach the nominal output power, and has a comparatively low cost. Its dynamic range spans, e.g., at least five orders of magnitude and is highly selective in that its capable of identifying and measuring the amount of water included in any media that may comprise, for example, aqueous solutions among a variety of other compositions.

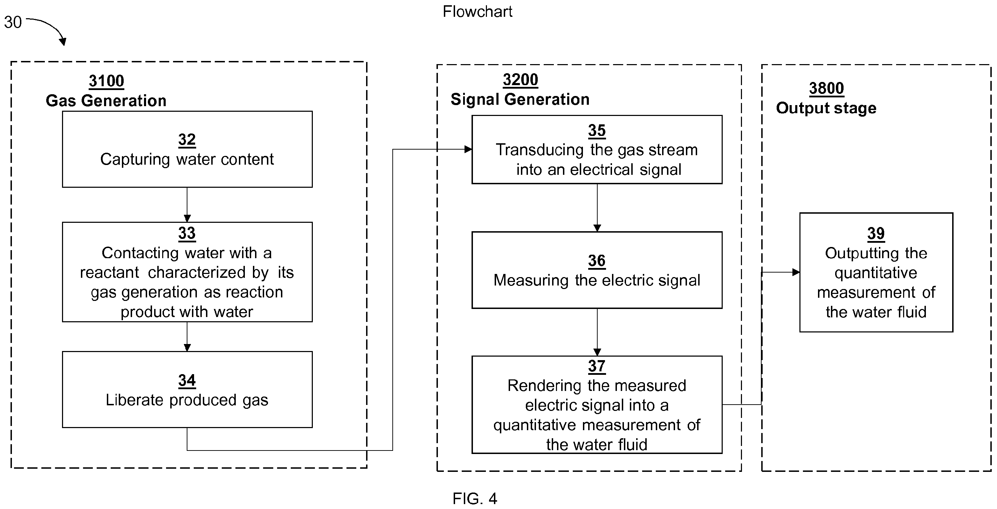

**FIG. 4** is a flowchart of the processing steps employed by the measurement device (e.g., water sensor) and can be divided into three stages, gas generation **3100,** signal generation **3200,** and output **3800.**

Specifically, in gas generation stage **3100,** water is captured at step **32** as a liquid, gas or vapor with a hydrophilic material, as noted above. In step **33,** the water is contacted with a reactant characterized by its gas generation properties as a reaction product. In step **34** the liberated gas is conveyed through a hydrophobic membrane **5** while filtering unreacted water and reacted CaH₂.

In stage **3200,** the liberated gas is transduced into an electrical signal at step **35** through any of the transducing element embodiments noted above. In step **36,** the signal is measured either as a current or a voltage in accordance with the type of transducing element employed. In step **37,** the measured signal is rendered into a quantitative measurement of the flow rate of water fluid in accordance with a given reaction conversion.

In the single-step output stage **3800,** the quantitative measurement is output at step **39,** for example, to a display screen.

## Claims

1. A measuring device (1) operably engageable with a skin surface portion of a skin surface of an animal body for measuring water content in a bodily media discharged by the skin surface, the measurement device being configured as a patch-like structure, wherein the measuring device (1) is **characterized by**:
a reactor (2) comprising a reactant gas donor (6), wherein the reactor (2) is configured to liberate a hydrogen stream having a stoichiometric equivalent to water in the media, the reactant gas donor (6) having an ability to liberate hydrogen gas upon reaction with water; and
a transducing element (8) configured to transduce the hydrogen stream into an electrical signal,
wherein the reactor (2) is configured such that the reactant gas donor (6) can be continuously subjected to flow of the bodily media.

2. The measuring device (1) of claim 1, further comprising circuitry (20, 21, 22, 23) that is configured to determine a value related to a quantity of water fluid in accordance with the electrical signal; and an output device configured to output the quantity of the water fluid.

3. The measuring device (1) of any one or more of the claims 1 to 2, wherein the reactant gas donor (6) is implemented as metallic or non-metallic hydride, wherein the metallic hydride is, for example, selected from the group consisting of MgH2, NaAIH4, LiAIH4, LiH, LiBH2, and LiBH4.

4. The measuring device (1) of claim 3, wherein the metallic hydride is implemented as CaH2.

5. The measuring device (1) of any one or more of claims 1 to 4, wherein the transducing element (8) is implemented as a fuel cell (8A, 8B) driven by the hydrogen stream.

6. The measuring device (1) of claim 5, wherein the fuel cell (8A, 8B) includes a proton-exchange membrane (PEM).

7. The measuring device (1) of any one or more of claims 1 to 6, wherein the transducing element (8) is implemented as porous dielectric (19) whose capacitance changes in accordance with a degree of permeation of the hydrogen stream, wherein the porous dielectric (19) is, for example, implemented as a zeolite.

8. The measuring device (1) of any one or more of claims 1 to 7, wherein the transducing element (8) is implemented as a pressure sensor configured to generate the electric signal responsively to a differential pressure generated by the hydrogen stream.

9. The measuring device (1) of any one or more of claims 1 to 8, wherein the transducing element (8) is implemented as an anemometer configured to generate the electric signal in accordance with gas flow of the hydrogen stream.

10. The measuring device (1) of any one or more of claims 1 to 9, configured such that the gas donor (6) makes direct or indirect contact with an animal body for measuring the water content contained in bodily media discharged by the animal body and, wherein, the bodily media comprises, for example, sweat.

11. The measuring device (1) of any one or more of the claims 1 to 10, further comprising a hydrophilic porous filter (4), wherein the gas donor (6) is disposed within and/or layered above the hydrophilic porous (4), wherein the hydrophilic porous filter (4) can be in fluid communication with the fluid source (10).

12. The measuring device (1) of any one or more of the claims 1 to 11, further comprising a water filter (5) upstream of the transducing element (6) to capture unreacted water.

13. The measuring device (1) of claim 12, wherein the water filter is a hydrophobic filter membrane

14. The measuring device (1) of any one or more of the claims 1 to 13, further comprising a fastener for operably coupling the measuring device (1) with the animal body.

## Patentansprüche

1. Messvorrichtung (1), die mit einem Hautoberflächenteil einer Hautoberfläche eines Tierkörpers in Eingriff gebracht werden kann, um den Wassergehalt in einem von der Hautoberfläche abgegebenen Körpermedium zu messen, wobei die Messvorrichtung als eine Patch-artige Struktur konfiguriert ist,
wobei die Messvorrichtung (1) **gekennzeichnet ist durch**:
einen Reaktor (2), der einen Reaktionsgasdonor (6) umfasst, wobei der Reaktor (2) so konfiguriert ist, dass er einen Wasserstoffstrom freisetzt, der ein stöchiometrisches Äquivalent zu Wasser in dem Medium aufweist, wobei der Reaktionsgasdonor (6) die Fähigkeit hat, bei der Reaktion mit Wasser Wasserstoffgas freizusetzen; und
ein Wandlerelement (8), das dafür konfiguriert ist, den Wasserstoffstrom in ein elektrisches Signal umzuwandeln,
wobei der Reaktor (2) so konfiguriert ist, dass der Reaktionsgasdonor (6) kontinuierlich dem Fluss des Körpermediums ausgesetzt werden kann.

2. Messvorrichtung (1) nach Anspruch 1, ferner umfassend eine Schaltung (20, 21, 22, 23), die so konfiguriert ist, dass sie in Übereinstimmung mit dem elektrischen Signal einen Wert bestimmt, der sich auf eine Menge eines wässrigen Fluids bezieht; und eine Ausgabevorrichtung, die so konfiguriert ist, dass sie die Menge des wässrigen Fluids ausgibt.

3. Messvorrichtung (1) nach einem oder mehreren der Ansprüche 1 bis 2, wobei der Reaktionsgasdonor (6) als metallisches oder nicht-metallisches Hydrid realisiert ist, wobei das metallische Hydrid beispielsweise aus der Gruppe ausgewählt ist, die aus MgH2, NaAlH4, LiAlH4, LiH, LiBH2 und LiBH4 besteht.

4. Messvorrichtung (1) nach Anspruch 3, wobei das Metallhydrid als CaH2 ausgeführt ist.

5. Messvorrichtung (1) nach einem oder mehreren der Ansprüche 1 bis 4, wobei das Wandlerelement (8) als Brennstoffzelle (8A, 8B) ausgeführt ist, die durch den Wasserstoffstrom angetrieben wird.

6. Messvorrichtung (1) nach Anspruch 5, wobei die Brennstoffzelle (8A, 8B) eine Protonenaustauschmembran (PEM) einschließt.

7. Messvorrichtung (1) nach einem oder mehreren der Ansprüche 1 bis 6, wobei das Wandlerelement (8) als poröses Dielektrikum (19) ausgeführt ist, dessen Kapazität sich in Abhängigkeit von einem Permeationsgrad des Wasserstoffstroms ändert, wobei das poröse Dielektrikum (19) beispielsweise als Zeolith ausgebildet ist.

8. Messvorrichtung (1) nach einem oder mehreren der Ansprüche 1 bis 7, wobei das Wandlerelement (8) als Drucksensor ausgeführt ist, der so konfiguriert ist, dass er das elektrische Signal als Reaktion auf einen durch den Wasserstoffstrom erzeugten Differenzdruck erzeugt.

9. Messvorrichtung (1) nach einem oder mehreren der Ansprüche 1 bis 8, wobei das Wandlerelement (8) als Anemometer ausgeführt ist, das so konfiguriert ist, dass es das elektrische Signal in Abhängigkeit vom Gasfluss des Wasserstoffstroms erzeugt.

10. Messvorrichtung (1) nach einem oder mehreren der Ansprüche 1 bis 9, die so konfiguriert ist, dass der Gasdonor (6) in direkten oder indirekten Kontakt mit einem Tierkörper kommt, um den Wassergehalt zu messen, der im Körpermedium enthalten ist, das von dem Tierkörper abgegeben wird, und wobei das Körpermedium beispielsweise Schweiß umfasst.

11. Messvorrichtung (1) nach einem oder mehreren der Ansprüche 1 bis 10, ferner umfassend einen hydrophilen porösen Filter (4), wobei der Gasdonor (6) innerhalb des hydrophilen porösen Filters (4) angeordnet und/oder darüber geschichtet ist, wobei der hydrophile poröse Filter (4) in Fluidverbindung mit der Fluidquelle (10) stehen kann.

12. Messvorrichtung (1) nach einem oder mehreren der Ansprüche 1 bis 11, ferner umfassend einen Wasserfilter (5) stromaufwärts des Wandlerelements (6), um nicht umgesetztes Wasser aufzufangen.

13. Messvorrichtung (1) nach Anspruch 12, wobei der Wasserfilter eine hydrophobe Filtermembran ist.

14. Messvorrichtung (1) nach einem oder mehreren der Ansprüche 1 bis 13, ferner umfassend ein Befestigungselement zur funktionellen Koppeln der Messvorrichtung (1) mit dem Tierkörper.

## Revendications

1. Dispositif de mesure (1) pouvant être mis en prise fonctionnellement avec une partie de surface de peau d'une surface de la peau d'un corps d'animal pour mesurer la teneur en eau dans un milieu corporel évacué par la surface de la peau, le dispositif de mesure étant configuré sous forme d'une structure de type pansement,
dans lequel le dispositif de mesure (1) est **caractérisé par** :
un réacteur (2) comprenant un donneur de gaz réactif (6), dans lequel le réacteur (2) est configuré pour libérer un flux d'hydrogène ayant un équivalent stoechiométrique à l'eau dans le milieu, le donneur de gaz réactif (6) ayant une aptitude à libérer de l'hydrogène gazeux lors de la réaction avec de l'eau, et
un élément transducteur (8) configuré pour convertir le flux d'hydrogène en un signal électrique,
dans lequel le réacteur (2) est configuré de sorte que le donneur de gaz réactif (6) peut être soumis de manière continue à l'écoulement du milieu corporel.

2. Dispositif de mesure (1) selon la revendication 1, comprenant en outre un circuit (20, 21, 22, 23) qui est configuré pour déterminer une valeur liée à une quantité d'eau fluide en fonction du signal électrique et un dispositif de sortie configuré pour délivrer la quantité d'eau fluide.

3. Dispositif de mesure (1) selon une ou plusieurs des revendications 1 à 2, dans lequel le donneur de gaz réactif (6) est mis en oeuvre sous forme d'un hydrure métallique ou non métallique, dans lequel l'hydrure métallique est sélectionné, par exemple, dans le groupe constitué de MgH2, NaAlH4, LiAlH4, LiH, LiBH2 et LiBH4.

4. Dispositif de mesure (1) selon la revendication 3, dans lequel l'hydrure métallique est mis en oeuvre sous forme de CaH₂.

5. Dispositif de mesure (1) selon une ou plusieurs des revendications 1 à 4, dans lequel l'élément transducteur (8) est réalisé sous forme d'une cellule à combustible (8A, 8B) alimentée par le flux d'hydrogène.

6. Dispositif de mesure (1) selon la revendication 5, dans lequel la cellule à combustible (8A, 8B) inclut une membrane échangeuse de protons (PEM).

7. Dispositif de mesure (1) selon une ou plusieurs des revendications 1 à 6, dans lequel l'élément transducteur (8) est réalisé sous forme d'un diélectrique poreux (19) dont la capacité varie en fonction d'un degré de perméation du flux d'hydrogène, dans lequel le diélectrique poreux (19) est réalisé, par exemple, sous forme d'une zéolite.

8. Dispositif de mesure (1) selon une ou plusieurs des revendications 1 à 7, dans lequel l'élément transducteur (8) est réalisé sous forme d'un capteur de pression configuré pour générer le signal électrique en réponse à une pression différentielle générée par le flux d'hydrogène.

9. Dispositif de mesure (1) selon une ou plusieurs des revendications 1 à 8, dans lequel l'élément transducteur (8) est réalisé sous forme d'un anémomètre configuré pour générer le signal électrique en fonction de l'écoulement gazeux du flux d'hydrogène.

10. Dispositif de mesure (1) selon une ou plusieurs des revendications 1 à 9, configuré de telle sorte que le donneur de gaz (6) vient en contact direct ou indirect avec un corps d'animal pour mesurer la teneur en eau contenue dans un milieu corporel évacué par le corps d'animal, dans lequel le milieu corporel comprend, par exemple, de la sueur.

11. Dispositif de mesure (1) selon une ou plusieurs des revendications 1 à 10, comprenant en outre un filtre poreux hydrophile (4), dans lequel le donneur de gaz (6) est disposé à l'intérieur et/ou superposé au-dessus du filtre poreux hydrophile (4), dans lequel le filtre poreux hydrophile (4) peut être en communication de fluide avec la source de fluide (10).

12. Dispositif de mesure (1) selon une ou plusieurs des revendications 1 à 11, comprenant en outre un filtre à eau (5) en amont de l'élément transducteur (6) pour capturer de l'eau n'ayant pas réagi.

13. Dispositif de mesure (1) selon la revendication 12, dans lequel le filtre à eau est une membrane filtrante hydrophobe.

14. Dispositif de mesure (1) selon une ou plusieurs des revendications 1 à 13, comprenant en outre un élément d'attache pour coupler fonctionnellement le dispositif de mesure (1) au corps d'animal.
